# EUROPEAN PATENT APPLICATION

(11) **EP 3 323 751 A1**
(43) Date of publication of application: **23.05.2018**
(21) Application number: 16824807.8
(22) Date of filing: 13.04.2016
(51) Int. Cl.: B65D 23/00, A61N 1/20

(54) **BOTTLE WITH ELECTROSTIMULATORY EFFECT**

(30) Priority: 13.07.2015 UA 201506955 U
(71) Applicant: Udovichenko, Vitalli Maksymovych, misto Mykolaiv 54020 (UA)
(72) Inventor: Udovichenko, Vitalli Maksymovych, misto Mykolaiv 54020 (UA)
(74) Representative: Zaboliene, Reda
(86) International application number: PCT/UA2016/000046
(87) International publication number: WO 2017/010965

(57) **Abstract**

A bottle with an electrostimulatory effect is equipped with an independent DC power source, disposed outside the internal cavity of the bottle, the outputs of which are connected to two electrodes, one of which is disposed on the inside surface of the bottle. The bottle has a neck, onto which a removable closure device can be mounted; an electrode, disposed on the inside surface of the bottle and configured in the form of a continuous electrically conductive circuit, said circuit encompassing the internal cavity of the bottle in the neck region; and a second electrode, also configured in the form of a continuous electrically conductive circuit, said circuit being disposed on the end of the neck such that when the closure device is in place, the electrode is isolated from the internal cavity of the bottle.

## Description

The invention relates to containers that have electrostimulation effect when consuming drinks that contained therein, and can be used in the bottle manufacturing for sports and energy drinks.

The impact on the human body by a fixed quantity of electric current impulses of a certain strength and voltage increases the efficiency of the organism and its reserves by stimulation of the human central nervous system and/or by achievement of the anti-sedative effect. A weak electrical impulse leads to a muscle contraction, increases their tonus, tonifies smooth muscles of blood vessels, stimulates blood flow - what causes the removing of the fatigue and the invigoration of the body. During electrical stimulation, impulses of electrical current stimulates the muscle structure and autonomic fibers that affect the course of metabolic processes in the muscles. Human muscles contract and perform some work influenced by these impulses, and the level of strain and duration of the muscles contraction depend on the human will and the electric current impulse characteristics. The muscles enhance their performance after electrical stimulation as reflected in an increase of aerobic potential, in an increase of glycolysis intensity and in the relevant ATP resynthesis mechanisms. According to the biochemical researches, + muscles and the entire body increases the energy potential and enzyme systems increases the activity, under the influence of the electrical stimulation. This enhances the oxidative processes and glycogen transformations in muscles, which becomes more available for enzymatic influences; lactic acid accumulation is prevented. Additionally, electrical stimulation influences not only on the muscles stimulation, but also on the entire body, and primarily on the central nervous system (CNS), on neurohumoral mechanisms of regulation functions through receptor apparatus. There is also the effect of electrical stimulation on the endocrine glands, as well as on the human immune system. Thus, the electrical stimulation leads not only to a violation of the neuromuscular structures, but also affects the trophic processes in muscles and all over the body which causes a non-specific basic functions increase in the entire organism.

It is known a bottle with an electrostimulatory effect is equipped with an independent DC power battery source, disposed outside the internal cavity of the bottle, the outputs of which are connected to two electrodes, one of which is disposed on the inside surface of the bottle, and the second on the outside surface of the bottle (Patent of invention RU Nº 2207161, A61M 35/00, A61N 1/04, A61N 1/30, A61N 1/38, published 27.06.2003).

However, the known bottle can only be used as a therapeutic device and it cannot be used for storing and transporting beverage therein; when using the known bottle, electric current passes through the large areas of the drinker's body, and there is no possibility to limit the area of its impact.

The technical problem of the invention consists in improvement of the bottle with an electrostimulatory effect is equipped with an independent DC power source, disposed outside the internal cavity of the bottle, the outputs of which are connected to two electrodes, one of which is disposed on the inside surface of the bottle, that the bottle has a neck, onto which a removable closure device can be mounted; an electrode, disposed on the inside surface of the bottle and configured in the form of a continuous electrically conductive circuit, said circuit encompassing the internal cavity of the bottle in the neck region; and a second electrode, also configured in the form of a continuous electrically conductive circuit, said circuit being disposed on the end of the neck such that when the closure device is in place, the electrode is isolated from the internal cavity of the bottle, and the DC power source is miniaturized in the form of a miniature battery or a miniature power element, or a miniature accumulator; such realization makes it possible to storage and to make transportation of the beverage in the bottle and to limit the area of influence of an electric current when drinking.

The bottle with an electrostimulatory effect has a neck, onto which a removable closure device can be mounted and equipped with an independent DC power source, disposed outside the internal cavity of the bottle, and the DC power source is miniaturized in the form of a miniature battery or a miniature power element, or a miniature accumulator etc., the outputs of DC power source - poles of a miniature battery or a miniature power element, or a miniature accumulator - connected to two electrodes, one of which is an electrode disposed on the inside surface of the bottle and configured in the form of a continuous electrically conductive circuit, said circuit encompassing the internal cavity of the bottle in the neck region, and the second electrode is also configured in the form of a continuous electrically conductive circuit, said circuit being disposed on the end of the neck such that when the closure device is in place, the electrode is isolated from the internal cavity of the bottle

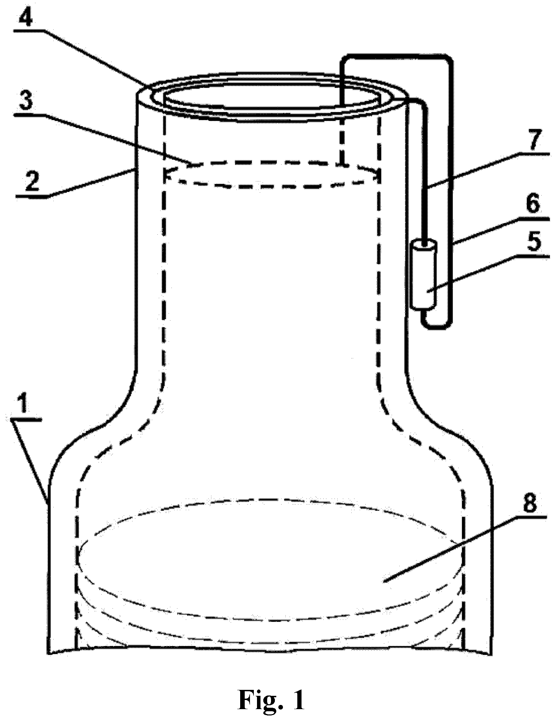

The figure, schematically, illustrates the bottle with an electrostimulatory effect. The bottle 1 with an electrostimulatory effect has a neck 2, onto which a removable closure device can be mounted (is not shown). The electrode 3 is disposed in the neck 2 region on the inside surface of the bottle 1, and configured in the form of a continuous electrically conductive circuit, said circuit encompassing the internal cavity of the bottle 1. The second electrode 4 is disposed on the end of the neck 2, and also configured in the form of a continuous electrically conductive circuit. For example, known plastic screw cover or cap for PET bottle can be used as a removable closure device, which pressurizes a pour opening of the bottle 1 when it is installed (screwing on) on the thread equipped in the neck 2 (see e.g. http://www.basketfood.org/packaging/m/19920_237_kryshki_dl.html or http://www.kz.all.biz/probki-kolpachki-i-drugie-upakovochnye-materialy-bgc498, or patent document EP0076778, B65D 41/04, B65D 41/34, published 13.04.1983, or patent document WO9602430, B65D 41/04, published 01.02.1996, or patent document WO9626121, B65D 41/04, published 29.08.1996 and others), wherein the design of the cover (cap) screwed on the neck 2 provides isolation of the electrode 4 disposed on the end of the neck 2 from the inside cavity of the bottle 1. Externally, the bottle 1 has a miniature independent DC power source 5 in the form of a miniature battery ("tablet" or "finger" or "mercurial finger"), a miniature power element or a miniature accumulator (see e.g. http://dic.academic.ru/dic.nsf/ruwiki/1037344 or http://dic.academic.ru/dic.nsf/ruwiki/659608, or http://trals.by/informacionnye-materialy/miniatyurnye-ehlementy-pitaniya/, or https://uk.wikipedia.org/wiki/ 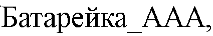 or, in particular, http://gamma.spb.ru/index.php/obuchenie/stati/varta/5-elementy-pttaniya-varta-microbattery-gmbh, or http://ohrana.ua/install/batarejki/) with appropriate parameters. The outputs of independent DC power source 5 - poles of a miniature battery or a miniature power element, or a miniature accumulator - respectively connected with the electrodes 3 and 4 via isolated conductors 6 and 7. The bottle 1 can be made of glass or PET. The electrodes 3 and 4 can be formed by any known method such as microelectronics or metal spraying, or by attaching the foil strips, in particular the electrode 4 may be formed as fixed on the end of the neck 2 ring of the foil. Installation of miniature independent DC power source 5, such as a miniature battery, can be accomplished in various ways; miniature independent DC power source 5 can be located in a housing and configured as a decorative element disposed near the neck 2, or can be located elsewhere (e.g., on the bottom of the bottle 1); conductors 6 and 7 can also serve for fixing the miniature independent DC power source 5, wherein it can be appropriate that they are completely or at least partially recessed into formed on surface the bottle 1 channels, or laid in the thickness of the material of the bottle 1 during its formation; possible variant of location of the miniature independent DC power source 5 e.g. a miniature battery, and conductors 6 and 7 in the thickness of the material of the bottle 1 during its formation, thus, in particular a miniature independent DC power source 5, e.g. a miniature battery can be located e.g. in the thickness of the bottom of the bottle 1.

Electrostimulation effect using the bottle 1 is obtained when consuming drinks that contained therein, directly from the neck 2. After removal of the closure device, e.g. after unscrewing of the plastic cover, which is provided by thread, the electrode 4 is exposed. A person's lips touches electrode 4, while drinking and when the bottle 1 is tilted, the beverage 8 that contains in it, completes the electrical circuit between the electrode 3 and the electrode 4, and therefore, person who is drinking, receives localized electrical discharge in mouth by the lips. Implementation of the electrode 4, configured in the form of a continuous electrically conductive circuit (e.g., foil ring) disposed on the end of the neck 2, allows electrode 4 to touch the lips in any position of the bottle 1. Implementation of the electrode 3, configured in the form of a continuous electrically conductive circuit (e.g., ring) provides the emergence of an electrical circuit at any amount of beverage 8 in the bottle 1 when it is tilted. The electrode 4 is securely isolated from the inside cavity the bottle 1 when the closure device is set (carefully screwed plastic cover), therefore the contact with a beverage 8 that contains in the bottle 1 and the electrode 4 is excluded, preventing discharge of the miniature independent DC power source 5 (in the form of a miniature battery or a miniature power element, or a miniature accumulator etc.) in any orientation of the bottle 1 in space during storage, transportation, and between consumption of the beverage 8.

The present method of electrical body stimulation is in a sub-threshold mode, which does not cause stimulated muscle contraction, but cause the feeling of an electrical current. Similar mode is used exclusively to affect multiple receptors and reflex effects on the processes in the most muscles and the whole body.

Suggested bottle with an electrostimulatory effect of the neuromuscular system can be used in the modern sports training system.

## Claims

1. A bottle with an electrostimulatory effect is equipped with an independent DC power source, disposed outside the internal cavity of the bottle, the outputs of which are connected to two electrodes, one of which is disposed on the inside surface of the bottle, **characterized in that** it has a neck, onto which a removable closure device can be mounted; an electrode, disposed on the inside surface of the bottle and configured in the form of a continuous electrically conductive circuit, said circuit encompassing the internal cavity of the bottle in the neck region; and a second electrode, also configured in the form of a continuous electrically conductive circuit, said circuit being disposed on the end of the neck such that when the closure device is in place, the electrode is isolated from the internal cavity of the bottle.

2. The bottle according to claim 1, **characterized in that** the DC power source is miniaturized in the form of a miniature battery or a miniature power element, or a miniature accumulator etc.
